# EUROPEAN PATENT APPLICATION

(11) **EP 3 495 376 A1**
(43) Date of publication of application: **12.06.2019**
(21) Application number: 17205408.2
(22) Date of filing: 05.12.2017
(51) Int. Cl.: C07K 1/34, C07K 1/18, C07K 1/16

(54) **IMPROVED CHROMATOGRAPHIC PROCESS FOR CELL CULTURE HARVEST**

(71) Applicant: ChromaCon AG, 8005 Zürich (CH)
(72) Inventor: WELDON, Richard, 8046 Zürich (CH); MÜLLER-SPÄTH, Thomas, 8050 Zürich (CH); BAVAND, Michael, 5600 Lenzburg (CH); AUMANN, Lars, 8049 Zürich (CH)
(74) Representative: Bremi, Tobias Hans

(57) **Abstract**

A method for purification of at least one protein target compound from a cell culture harvest comprising said at least one protein target compound as well as impurities, said method comprising the following steps in given order:
a) at least one step for removal of insoluble impurities from the initial cell culture harvest;
b) at least one further initial cell culture harvest pre-treatment step for removal of anionic soluble impurities from the cell culture harvest;
c) separation of the at least one protein target compound from the cell culture harvest by means of affinity chromatography,
with the proviso that the order steps a) and b) can be reversed or that the steps a) and b) are carried out in one device,
and with the proviso that further pre-treatment steps can be carried out before the terminal step c) of affinity chromatography separation.

## Description

### TECHNICAL FIELD

The present invention relates to an improved chromatographic process in particular for the purification of protein compounds from cell culture harvest. The present invention in particular relates to a method involving pre-treatment and subsequent chromatographic purification as well as to uses of the pretreatment.

### PRIOR ART

Affinity chromatography is a well-established technique in the manufacturing of biopharmaceuticals. It is used for primary capture of therapeutic proteins following cell culture harvest and removal of insoluble impurities in many downstream processes. It is also used for purification of plasma proteins. Affinity chromatography is highly selective for the target compounds and therefore generally provides excellent impurity clearance. In the processing of proteins containing Fc domains such as IgGs and Fc fusion proteins, Protein A affinity chromatography is used in the majority of cases today.

Due to the elaborate ligands that provide the high specificity and binding strength for the target compounds, affinity chromatography materials are expensive and less stable than simpler ligands, e.g. ligands for cation exchange (CEX) or anion exchange (AEX) chromatography.

Before primary capture by affinity chromatography, the feed streams containing the product compounds of interest are generally pretreated for initial removal of particulate material such as cell debris originating from the cell culture process and insoluble aggregates to avoid clogging of the subsequent chromatography steps. This step for removal of insoluble impurities is also referred to as "clarification" and the treated material is referred to as "clarified harvest".

The pre-treatment normally involves filtration steps, centrifugation steps, flocculation steps, acoustic separation steps or precipitation steps, and combinations of the aforementioned techniques. Filtration techniques include depth filtration, absolute filtration and ultrafiltration. Frequently depth filtration steps with two different cut-offs such as 0.45 µm and 0.22 µm cut-off are combined. Typically filtration pre-treatment also contributes to removal of soluble process-related impurities such as host cell proteins (HCPs) to some extent.

Recently, filters combining depth filtration with ion exchange functionalities have been introduced with the aim to improve the impurity clearance capabilities of traditional depth filters.

As mentioned above, affinity materials are expensive. It is therefore of high interest in chromatography processes to operate at a high affinity material capacity utilization, i.e. a high load of target compound per affinity material volume. At the same time it is of high interest to operate at high throughput, i.e. high productivity.

Today, affinity chromatography is predominantly used in the format of particle-based affinity resins in packed bed columns. Particle-based affinity resins provide high capacity for the target compounds, however this format is limited in throughput as backpressure increases in packed beds with increasing flow rate. The pressure rating of the columns and the equipment used sets an upper boundary for the flow rate in the capture step and thus limits the productivity. With large particle resins the backpressure of the packed bed can be reduced, however due to mass transfer effects and dispersion, the dynamic capacity of a column with larger particles is lower compared to a resin with smaller particles. Thus the breakthrough profile of the product compound becomes increasingly diffuse as the flow rate increases, leading to earlier breakthrough of product in the capture step and reduced resin capacity utilization. Thus an increased productivity can only be obtained at the cost of decreased capacity utilization and vice versa.

Employing additional columns in the capture step can alleviate this trade-off. So called periodic counter-current (PCC) processes use a second adsorber connected in series with a first adsorber during the loading phase of a capture step. Thereby the first adsorber can be overloaded such that its capacity is fully utilized and the target compound breaking through is captured on the second adsorber. The loading is performed at the maximum possible flow rate. This way high capacity utilization and increased productivity can be achieved simultaneously. However a flow rate limitation persists if particle-based adsorbers (packed bed columns) are used. Alternative adsorber formats have been suggested, allowing operation at significantly higher flow rates than traditional packed bed chromatography. These formats include membrane or fiber-based adsorbers. Also packed-beds with internal supporting structures based on inorganic compounds or meshes have been suggested. Generally membrane adsorbers contain polymeric layers with porous structures which are arranged in a housing to maximize binding and throughput. Membranes allow a much faster mass transfer due to convective flow as opposed to particle based adsorbers where the main mass transfer contribution is by diffusion through a porous structure which is far more branched and tortuous than that of a membrane. Through this pore structure, particle-based resins are capable of providing much larger internal surface areas than membrane-based adsorbers. Thus, despite having inferior flow rate capabilities, particle-based resins have remained the standard due to their higher capacities for product binding.

Only recently, membrane-based adsorbers for affinity chromatography providing comparable capacity as particle-based adsorbers have been introduced, allowing much higher flow rates.

Despite being based on convective mass transfer, most membrane-based adsorbers display a diffuse breakthrough curve similar to the curve obtained with particle-based adsorbers when loaded with feed material containing product, which is due to hydrodynamic effects such as dispersion and back-mixing within the membrane-based adsorber housing. Therefore PCC processes are also advantageous when combined with membrane-based adsorbers (or fibrous materials).

### SUMMARY OF THE INVENTION

It is the object of the present invention to propose an improved chromatographic purification method, in particular for separating the protein target compound from a cell culture harvest.

The invention more specifically relates to a pre-treatment method for a process using affinity-based capture materials. Affinity chromatography is a method of separating biochemical mixtures based on a highly specific interaction between antigen and antibody, enzyme and substrate, or receptor and ligand. It is used for purifying biological molecules within a mixture by exploiting molecular properties. Biological macromolecules, such as enzymes and other proteins, interact with other molecules with high specificity through several different types of bonds and interactions. Such interactions including hydrogen bonding, ionic interaction, disulfide bridges, hydrophobic interaction, and more. The high selectivity of affinity chromatography is caused by allowing the desired molecule transported in the liquid phase in a mixture with undesired impurities to interact with the stationary phase, carrying immobilized ligands for the desired molecule, and be bound within the column in order to be separated from the undesired material which will not interact and elute first. The impurity molecules no longer needed are first washed away with a buffer while the desired target proteins are let go in the presence of the eluting solvent (of higher salt concentration or the like, different from the buffer). The process creates a competitive interaction between the desired protein and the immobilized stationary molecules, which eventually lets the now highly purified proteins be released. Affinity chromatography capture materials thus allow highly selective purification process with essentially no overlapping fractions, retention of the target molecules on the stationary phase being almost completely selective under a first set of liquid conditions, namely under conditions of the feed (the feed being the mixture of the target molecules and the impurities) and under conditions of a correspondingly adapted buffer, while under a second set of liquid conditions, using a so-called eluting liquid, typically having a different salt concentration, a different pH or the like and being different from the conditions during the feed and the buffer, the target molecules are released from the stationary phase.

Surprisingly, it was found that running cell culture harvest, despite having been clarified by means of depth filtration and additional absolute filtration led to the increase of backpressure during the loading of affinity adsorbers. This is surprising because all insoluble impurities which would be usually associated with effects like clogging and fouling had been removed by the previous clarification steps. The backpressure increase was present in both particle-based and membrane-based adsorbers however it was much stronger in the case of membrane-based adsorbers. In the latter case the backpressure was only partially reversible through a cleaning protocol. Due to the increased backpressure it was not possible to operate the membrane-based adsorbers in a periodic countercurrent (PCC) process, which includes multiple load cycles, without exceeding the pressure rating of the membrane-based adsorber device.

Moreover it was surprisingly found that by further pretreating the clarified harvest by running it through a membrane adsorber with anion exchange functionality the backpressure increase in the subsequent loading steps of single adsorber and PCC processes could be avoided to a large extent. It was confirmed that removal of anionic species was required to achieve this, as clarified cell culture harvest treated with a cation exchange (CEX) membrane instead of an anion exchange (AEX) membrane showed a similar back-pressure increase as clarified cell culture harvest that had not been pre-treated. Thus it can be concluded that with a pre-treatment including anion exchange functionality, in addition to an initial clarification, it was possible to remove soluble contaminants that, if remaining in the clarified harvest, would have led to a backpressure increase of the affinity adsorbers, strongly limiting their lifespan.

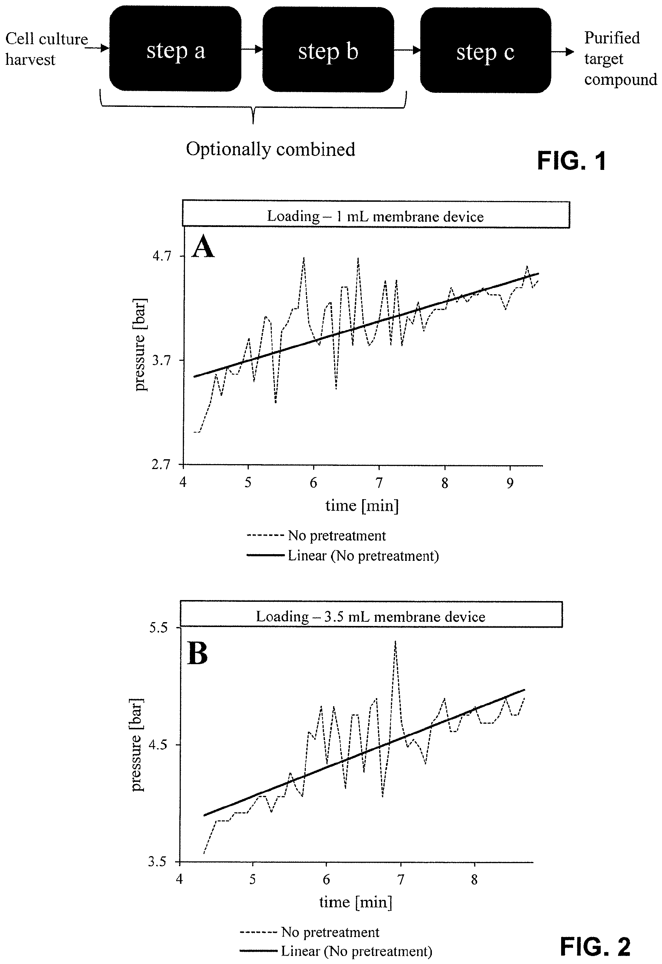

It is thus one object of the present invention to propose a method for purification of the protein target compound from cell culture harvest according to Fig 1, comprising:
a) at least one step for removal of insoluble impurities
b) at least one further pretreatment step for removal of anionic soluble impurities to avoid a backpressure increase in the subsequent affinity step
c) capture of the protein target compound of interest by means of affinity chromatography.

Step a) may include filtration, centrifugation, acoustic separation, flocculation or precipitation steps, and combinations of these aforementioned techniques. Filtration techniques include depth filtration, absolute filtration and ultrafiltration.

Steps a) and b) may be combined within one step and one device by using filters with anion exchange functionality.

More generally speaking, the present invention proposes a method for purification of at least one protein target compound from a cell culture harvest comprising said at least one protein target compound as well as impurities, said method comprising the following steps in given order:
a) at least one step for removal of insoluble impurities from the initial cell culture harvest;
b) at least one further initial cell culture harvest pre-treatment step for removal of anionic soluble impurities from the cell culture harvest;
c) separation of the at least one protein target compound from the cell culture harvest by means of affinity chromatography,
with the proviso that the order steps a) and b) can be reversed or that the steps a) and b) are carried out in one device, for example in one step, and with the proviso that further pre-treatment steps can be carried out before the terminal step c) of affinity chromatography separation.

The pre-treatment step b) typically leads to a reduction of the specific backpressure increase (ΔPs, how the specific backpressure increase of the chromatographic adsorbers, can be derived for a particular setup is defined further below) in the subsequent affinity step c) by at least 50%, preferably by at least 60%, or by at least 70%, most preferably by at least 80%, in each case relative to the specific backpressure increase without such pre-treatment step b).

Alternatively expressed in absolute figures, in many setups the pre-treatment step b) typically leads to a reduction of the specific backpressure increase (ΔPs) in the subsequent affinity step c) by at least 0.02 bar*(mL adsorbent)/(mL feed), preferably by at least 0.03 bar*(mL adsorbent)/(mL feed), most preferably by least 0.04 bar*(mL adsorbent)/(mL feed).

According to a first preferred embodiment, said affinity chromatography separation step c) works with at least two affinity chromatography adsorbers and comprises at least two different phases (IC, B),
at least one interconnected phase (IC), in which two affinity chromatography adsorbers are interconnected in that the outlet of an upstream adsorber is fluidly connected to the inlet of a downstream adsorber, and
at least one batch phase (B), in which at least one affinity chromatography adsorber is not fluidly connected to the others and in which the at least one protein target compounds are recovered in purified form from a disconnected adsorber.

The step a) for removal of insoluble impurities from the initial cell culture harvest, or at least one module of a corresponding pre-treatment device, may include filtration, centrifugation, flocculation steps, acoustic separation, or precipitation steps, or a combination of these aforementioned techniques and the filtration techniques include depth filtration, absolute filtration or ultrafiltration.

The steps a) for removal of insoluble impurities from the initial cell culture harvest, as well as the step b) for removal of anionic soluble impurities, can also be carried out by the same medium, preferably in one step, in that the method or at least one module of a corresponding pre-treatment device, includes filtration, centrifugation, flocculation steps, acoustic separation, or precipitation steps, or a combination of these aforementioned techniques and wherein the filtration techniques include depth filtration, absolute filtration or ultrafiltration, and wherein the corresponding method involves at the same time removal of anionic soluble impurities from the cell culture harvest.

Preferably the method in which the steps a) and b) are carried out concomitantly involves a filter carrying positive stationary charges.

According to yet another preferred embodiment, step b) is performed using a chromatographic sorbent for removal of anionic soluble impurities and preferably the sorbent is based on membranes, packed beds of particles, inorganic compounds, monoliths, fibers or combinations thereof.

According to yet another preferred embodiment, step b) uses an anion exchange functionality, or is based on hydroxyapatite or fluoroapatite.

The further pre-treatment step b. may include particle or membrane-based adsorbers, whereby the use of membrane-based adsorbers is preferred due to their high flow rate capabilities, reducing the time required for the treatment.

In a multicolumn setup anion exchange adsorbers for step b. may be run in the same chromatography equipment as the adsorbers used for affinity capture in step c.

The affinity capture step c. may include a cyclic single adsorber capture process or a multi-adsorber process. The multi-adsorber process may be operated with at least one phase during which two adsorbers are interconnected and the upstream adsorbers is loaded such that the product breaking through form the upstream adsorber is captured in the second adsorber.

Step c) can be performed using an affinity chromatography sorbent based on membranes, packed beds of particles, monoliths, fibers, inorganic compounds, or combinations thereof. Step c) can furthermore be performed using an affinity chromatography sorbent for protein A, protein G, protein L, metal ion affinity chromatography, organic ligands, heparin, or affinity chromatography using other proteinaceous or peptidic ligands.

In the affinity chromatography step c) it is possible to use at least two affinity chromatography adsorbers, preferably of the same type, preferably exactly two affinity chromatography absorbers.

In this case preferably the affinity chromatography step c) comprises at least two cycles wherein each cycle includes at least one interconnected phase (IC) wherein the outlet of an upstream adsorber is connected to the inlet of a downstream adsorber, as well as at least one batch phase (B) in which the adsorbers are not fluidly connected, and wherein preferably the affinity chromatography step c) is operated over at least two cycles.

According to a particularly preferred embodiment, in the affinity chromatography step c) a method is used as described in WO 2014/166799 the disclosure of which as concerns step c) is expressly included into this disclosure.

According to a preferred embodiment therefore the method is preferably carried out using at least two affinity chromatography adsorbers, and comprises at least four different steps (IC1, B1, IC2, B2) in given order, within one cycle to be carried out at least once, preferably a plurality of times, preferably using only two affinity chromatography adsorbers:
a first batch step (B1), wherein during a batch timespan (t_{B}) said adsorbers are disconnected and
   a first adsorber is loaded with feed with the cell culture harvest pretreated in steps a) and b) via its inlet, using a first flow rate (Q_{feed,B}) and its outlet is directed to waste, and
   from a second adsorber the at least one protein target compound is recovered via its outlet and subsequently the second adsorber is regenerated;
a first interconnected step (IC1), wherein the outlet of the first adsorber is connected to the inlet of the second adsorber during an interconnected timespan (tic),
   the first adsorber is loaded beyond its dynamic breakthrough capacity with feed via its inlet using a second flow rate (Q_{feed,IC}) which is the same or larger than the first flow rate (Q_{feed,B}), and
   the outlet of the second adsorber is directed to waste,
   and wherein during a subsequent washing timespan (t_{wash,IC}) which is larger or equal to 0 s
      the outlet of the first adsorber is connected to the inlet of the second adsorber,
      the first adsorber is loaded with solvent and/or buffer which is free from feed material, and
      the outlet of the second adsorber is directed to waste,
a second batch step (B2) analogous to the first batch step (B1) but with exchanged adsorber positions, such that the first adsorber of the first batch step (B1) performs the tasks of the second adsorber of the first batch step (B1), and the second adsorber of the first batch step (B1) performs the tasks of the first adsorber of the first batch step (B1);
a second interconnected step (IC2), analogous to the first interconnected step (IC1) but with exchanged adsorber positions, such that the upstream adsorber of the first interconnected step (IC1) is the downstream adsorber of the second interconnected step (IC2), and the downstream adsorber of the first interconnected step (B1) is the upstream adsorber of the second interconnected step (IC2).

The protein target compound is preferably a monoclonal antibody, bispecific antibody, antibody fragment, multispecific antibody framework, antibody scaffold, or fusion protein.

Also for the abovementioned affinity capture steps c), the use of membrane based adsorbers is preferred due to their high flow rate capabilities, however the described method is also applicable to particle-based resins. The affinity material utilized preferably uses proteinaceous or peptidic ligands such as protein A, protein G, protein L.

Step b) may include a regeneration step and the device can be operated in a cyclic manner

The above process including steps can, according to a preferred embodiment, be followed by a further separation step as defined in PCT/EP 2017/067190, the disclosure of which is expressly included as concerns such an additional purification step into the present application.

Correspondingly, according to a preferred embodiment, step c) is followed by a virus inactivation step and by a method using at least two chromatographic non-affinity adsorbers as chromatographic stationary phase, grouped into only one first adsorber section and one second adsorber section,
wherein if an adsorber section comprises more than one chromatographic adsorber these are permanently connected in series,
wherein the first adsorber section has a first adsorber section inlet and a first adsorber section outlet, and the second adsorber section has a second adsorber section inlet and a second adsorber section outlet,
the method comprising the following steps in order:
a. a first interconnected step (IC1a), wherein the first adsorber section outlet is connected to the second adsorber section inlet during an first interconnected timespan (t_{ICa}), wherein the first adsorber section (1) is loaded via the first adsorber section inlet with feed mixture and wherein product is collected from the second adsorber section outlet;
b. a second interconnected step (IC1b), wherein the first adsorber section outlet is connected to the second adsorber section inlet during a second interconnected timespan (t_{ICb}), wherein the first adsorber section is loaded via the first adsorber section inlet with a first washing buffer to transfer product unbound in the first absorber section into the second adsorber section and wherein the stream exiting the first adsorber section outlet is either diluted inline before entering the second adsorber section inlet or is supplemented with feed mixture
   and wherein product is collected from the second adsorber section outlet;
c. a first batch step (B1), wherein during a batch timespan (t_{B}) said first and second adsorber sections are disconnected and
   wherein the first adsorber section is cleaned and regenerated to remove impurities and the first adsorber section outlet is directed to waste and wherein the second adsorber section inlet is
   either loaded with a second washing buffer
   or loaded with feed mixture
   and product is collected from the second adsorber section outlet;
d. a third interconnected step (IC2a), wherein the first adsorber section performs tasks of the second adsorber section in the first interconnected step, and the second adsorber section performs tasks of the first adsorber section in the first interconnected step;
e. a fourth interconnected step (IC2b), wherein the first adsorber section performs tasks of the second adsorber section in the second interconnected step, and the second adsorber section performs tasks of the first adsorber section in the second interconnected step;
f. a second batch step (B2), wherein the first adsorber section performs tasks of the second adsorber section in the first batch step, and the second adsorber section performs tasks of the first adsorber section in the first batch step.

Furthermore the present invention relates to the use of a pre-treatment for removal of anionic soluble impurities from a cell culture harvest for reducing the back pressure in a subsequent affinity step capture separation.

Such a use for purification of at least one protein target compound from a cell culture harvest comprising said at least one protein target compound as well as impurities, the following steps can be carried out in given order:
a) at least one step for removal of insoluble impurities from the initial cell culture harvest;
b) at least one further initial cell culture harvest pre-treatment step for removal of anionic soluble impurities from the cell culture harvest;
c) separation of the at least one protein target compound from the cell culture harvest by means of affinity chromatography,
with the proviso that the order steps a) and b) can be reversed or that the steps a) and b) are carried out in one device in one step, and with the proviso that further pre-treatment steps can be carried out before the terminal step c) of affinity chromatography separation.
The removal of anionic soluble impurities can be carried out by anionic exchange chromatography (AEX), preferably by using a packed particle bed or a membrane anionic exchange chromatography device.
Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic representation of the method for purification of the target compound;
- Fig. 2: in A - shows the backpressure increase recorded during the loading of clarified CHO cell culture harvest ("feed") during a single-adsorber method using a membrane-based 1 mL Protein A capture device (GORE Inc., DE, USA), and a linear fit of the data;
in B - shows the backpressure increase recorded during the loading of clarified CHO cell culture harvest during a single-adsorber method using a membrane-based 3.5 mL Protein A capture device (GORE Inc., DE, USA), and a linear fit of the data;
in C - shows the backpressure increase recorded during the loading of clarified CHO cell culture harvest during a single-adsorber method using an Eshmuno A 5.0 mL Protein A column (Millipore, Germany), and a linear fit of the data;

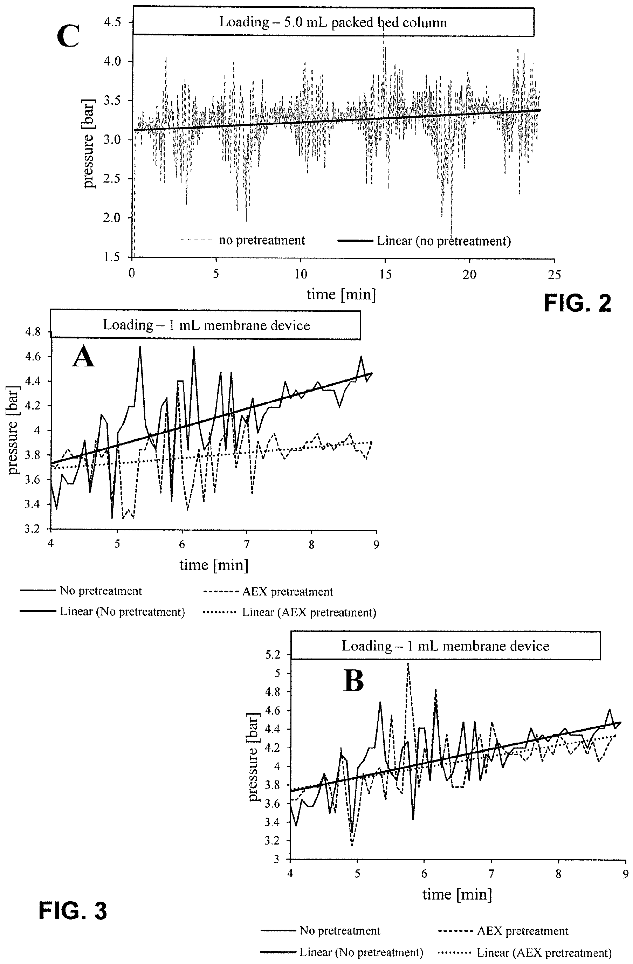

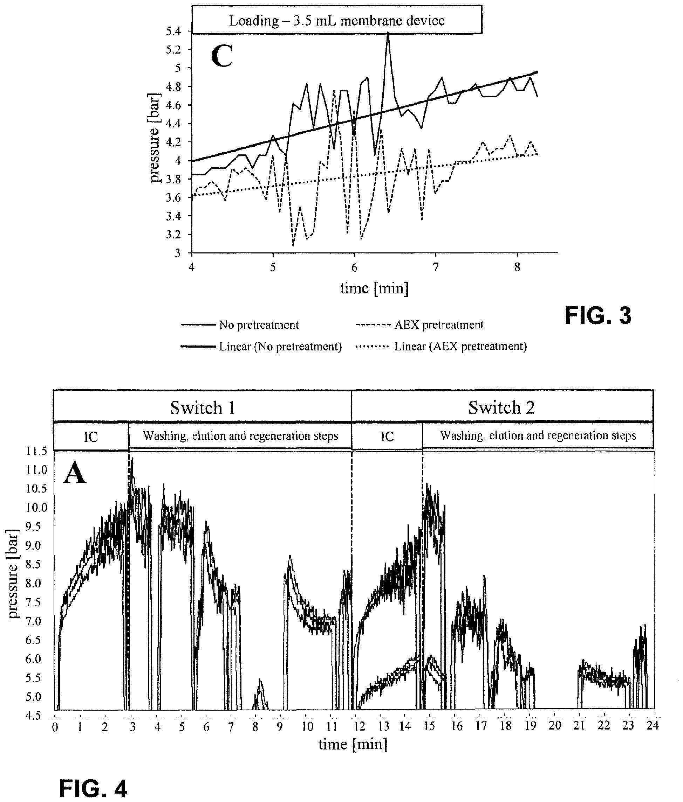

- Fig. 3: in A - shows that the backpressure increase in Fig. 2A can be significantly reduced by pre-treating clarified CHO cell culture harvest with an AEX membrane before loading the 1 mL Protein A capture device;
in B - shows that the backpressure increase in Fig. 2A cannot be prevented by pre-treating clarified CHO cell culture harvest with a CEX membrane before loading the 1 mL Protein A capture device;
in C - shows that the backpressure increase in Fig. 2B can be significantly reduced by pre-treating clarified CHO cell culture harvest with an AEX membrane before loading the 3.5 mL Protein A capture device;

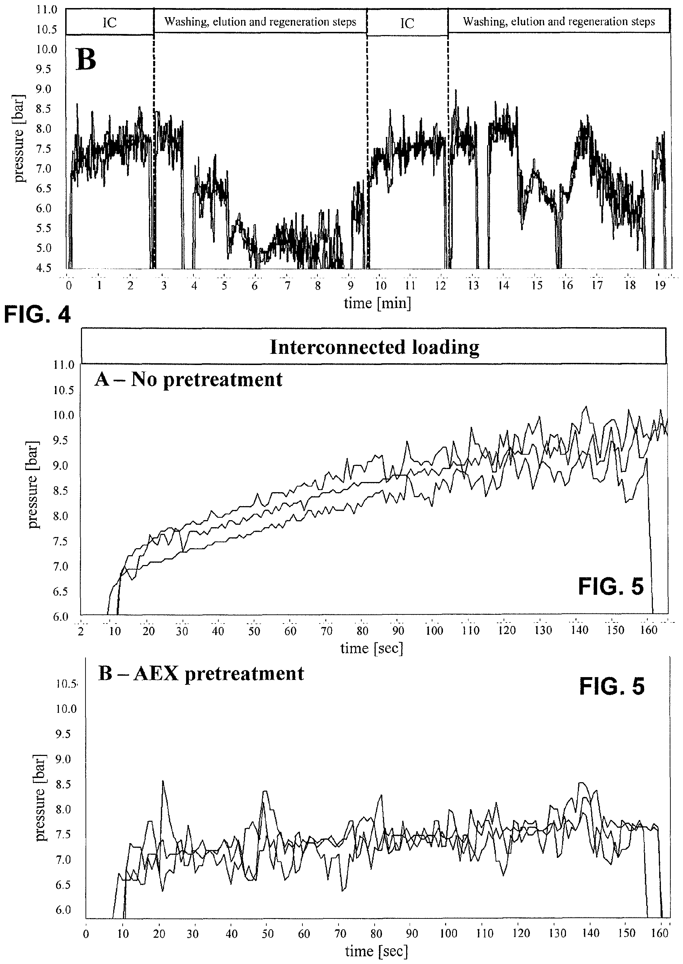

- Fig. 4: in A - shows the backpressure profiles of a PCC process using two 3.5 mL Protein A capture devices (GORE Inc., DE, USA) loaded with untreated clarified CHO cell culture harvest - Pressure readings for 3 consecutive cycles are overlaid;
in B - shows the backpressure profiles of a PCC process using two 3.5 mL Protein A capture device loaded with AEX pre-treated clarified CHO cell culture harvest - Pressure readings for 3 consecutive cycles are overlaid
in each case: IC: interconnected step; x-axis: time in min, y-axis: PI [bar], P2 [bar], P3 [bar];

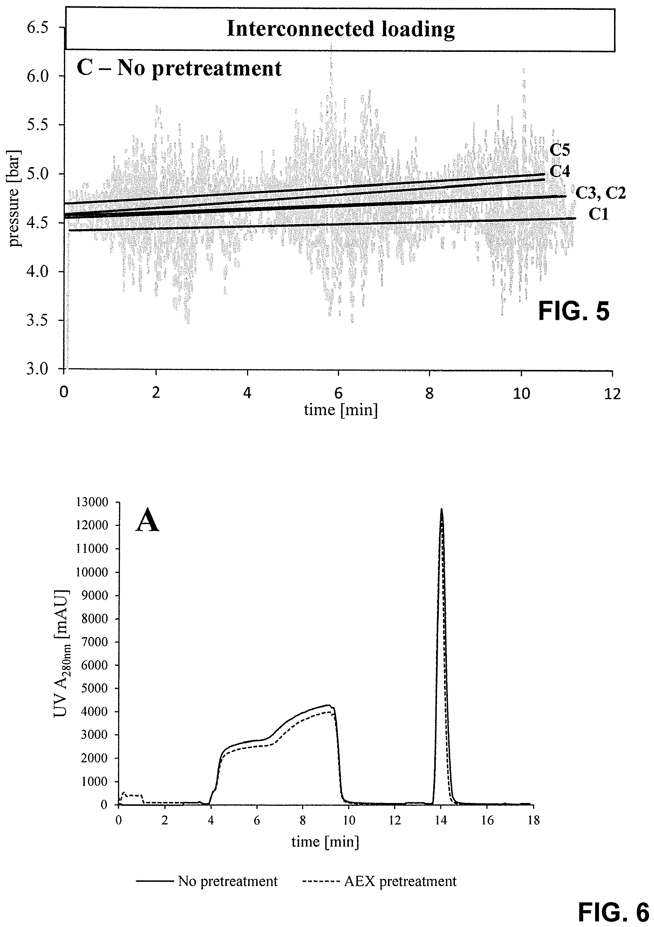

- Fig. 5: in A - shows a zoom of fig. 4A showing the first interconnected loading phase of the PCC process;
in B - shows a zoom of fig. 4B showing the first interconnected loading phase of the PCC process;
in C - shows a zoom of the first interconnected loading phase of a PCC process run using two Eshmuno A 5.0 mL Protein A columns (Millipore, Germany) - Pressure readings for 5 consecutive cycles C1-C5 are overlaid and the linear fits of the pressure curves are provided;

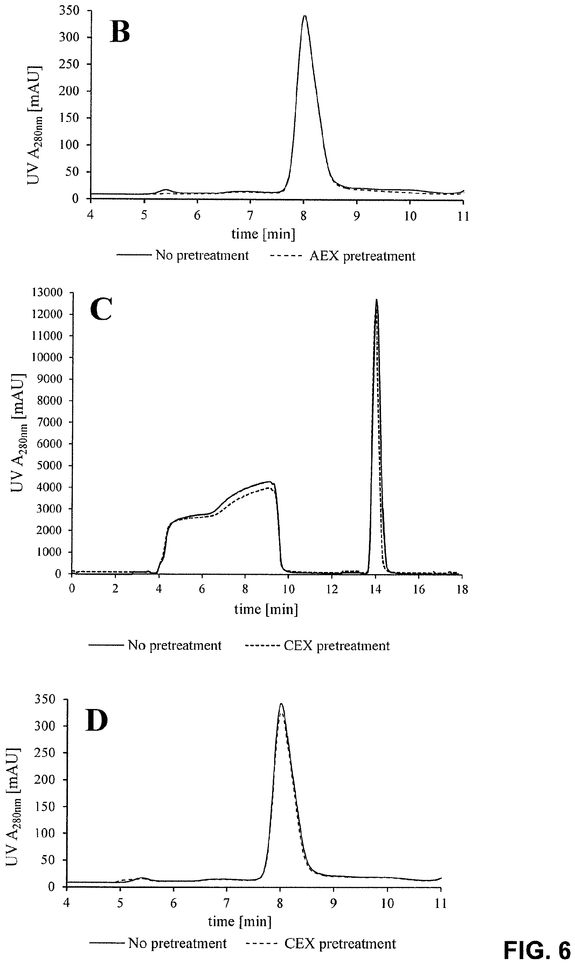

- Fig. 6: in A - shows the UV A 280 nm signal recorded during the loading of clarified CHO cell culture harvest during a single-adsorber run using a 1 mL Protein A capture device (GORE Inc., DE, USA);
in B - shows the SE-HPLC chromatogram of the non-treated vs. the AEX treated feed;
in C - shows the UV A 280 nm signal recorded during the loading of CHO cell supernatant during a single-adsorber run using a 1 mL Protein A capture device;
in D - shows the SE-HPLC chromatogram of the non-treated vs. the CEX treated feed.

### DESCRIPTION OF PREFERRED EMBODIMENTS

**Example 1:** A breakthrough curve was generated for two Protein A membrane-based capture devices (GORE Inc., DE, USA) with 1 mL and 3.5 mL adsorbent volume, respectively. The 1 mL Protein A membrane-based device had a 1.1 cm inner diameter and 0.5 cm length. The 3.5 mL Protein A membrane-based device had a 3 cm inner diameter and 0.5 cm length. Table 1 & Table 2 shows the operating conditions used to generate the curves for the 1 mL and 3.5 mL devices respectively. The feed used to generate the breakthrough curves was a CHO cell culture harvest clarified by depth filtration and the concentration of IgG in the feed was 5.0 g/L. Prior to the run, all feed and buffers were filtered using a Millipore, Durapore 0.45 µm PVDF membrane to prevent backpressure increases due to fouling of the Protein A membrane-based adsorbers with particulates.

The backpressure during the feed step of the batch run for each membrane device is shown in **Figure 2****.** Figure 2A shows results for the 1 mL device and Figure 2B shows the results for the 3.5 mL device. The membrane-based capture devices of both dimensions demonstrated increased backpressure as loading proceeded, so this observation is independent of device scale.

The specific backpressure increase of the chromatographic adsorbers, ΔPs, can be derived through the following procedure:
The specific loading Lₛ of the device can be expressed in terms of load of feed V_{feed} [mL feed] per adsorbent volume V_{ads} [mL adsorbent]: Lₛ = V_{feed}/V_{ads} [mL feed]/[mL adsorbent]. The load of feed V_{feed} can be determined by multiplying the feeding period t_{feed} [min] and the feed flow rate Q_{feed} [mL feed / min]: V_{feed} = t_{feed}*Q_{feed}.

The specific backpressure increase ΔPs can then be quantified as ΔPs = ΔP/Lₛ with the units [bar] x [mL adsorbent]/[mL feed].

Thus the specific backpressure increase is ΔPs = (ΔP*V_{ads})/(t_{feed}*Q_{feed}).

The values ΔP and t_{feed} can be determined from the linear fits of the pressure curves where ΔP is the pressure increase within a certain feeding period t_{feed}. The adsorbent volume V_{ads} and the flow rate Q_{feed} are fixed parameters.

In example A the specific backpressure increase is ΔPs = 0.051 bar*(mL adsorbent)/(mL feed) and in example B it is ΔPs = 0.075 bar*(mL adsorbent)/(mL feed) .

Moreover, a backpressure increase has been observed, albeit to a lower degree, for Protein A resins. Figure 2C shows the result for a particle-based protein A adsorber (Eshmuno A column, Merck-Millipore, column 0.8 cm inner diameter x 10 cm bed height). The specific backpressure increase is ΔPs = 0.023 bar*(mL adsorbent)/(mL feed). Protein A capture steps were carried out using a Contichrom CUBE Combined 30 system (ChromaCon AG, Switzerland).

**Table 1: Batch mode operating parameters for the 1mL membrane-based device**

| **Phase** | **step** | **Q** | **buffer** | **t** |
|---|---|---|---|---|
| **[-]** | **[-]** | **[mL/min]** | [-] | **[min]** |
| **1mL Batch** | equilibrate | 3 | 25 mM Phosphate, 150 mM NaCl pH 7.0 | 3.33 |
| | feed | 3 | CHO cell supernatant - mAb (5g/L) | 5.33 |
| | wash | 3 | 25 mM Phosphate, 150 mM NaCl pH 7.0 | 3.33 |
| | line wash | 3 | 100 mM Citrate, pH 3.5 | 0.66 |
| | elute | 3 | 100 mM Citrate, pH 3.5 | 3.33 |
| | neutralization | 3 | 25 mM Phosphate, 150 mM NaCl pH 7.0 | 0.66 |
| | strip | 1 | 100 mM NaOH | 5 |
| | re-equilibration | 3 | 25 mM Phosphate, 150 mM NaCl pH 7.0 | 2.66 |

**Table 2: Batch mode operating parameters for the 3.5mL membrane-based device**

| **Phase** | **step** | **Q** | **buffer** | **t** |
|---|---|---|---|---|
| **[-]** | **[-]** | **[mL/min]** | **[-]** | **[min]** |
| **Batch** | equilibrate | 10.5 | 25 mM Phosphate, 150 mM NaCl pH 7.0 | 3.33 |
| | feed | 10.5 | CHO cell supernatant - mAb (5g/L) | 5.33 |
| | wash | 10.5 | 25 mM Phosphate, 150 mM NaCl pH 7.0 | 3.33 |
| | line wash | 10.5 | 100 mM Citrate, pH 3.5 | 0.66 |
| | elute | 10.5 | 100 mM Citrate, pH 3.5 | 3.33 |
| | neutralization | 10.5 | 25 mM Phosphate, 150 mM NaCl pH 7.0 | 0.66 |
| | strip | 3.5 | 100 mM NaOH | 5 |
| | re-equilibration | 10.5 | 25 mM Phosphate, 150 mM NaCl pH 7.0 | 2.66 |

**Table 3: Batch mode operating parameters for the 5mL Eshmuno A column**

| **Phase** | **step** | **Q** | **buffer** | **t** |
|---|---|---|---|---|
| **[-]** | **[-]** | **[mL/min]** | **[-]** | **[min]** |
| **Batch** | equilibrate | 2.5 | 25 mM Phosphate, pH 7.0 | 10 |
| | feed | 2.5 | CHO cell supernatant - mAb (5g/L) | 24 |
| | Wash 1 | 2.5 | 25 mM Phosphate, pH 7.0 | 10 |
| | Wash 2 | 2.5 | 25 mM Phosphate, 1M NaCl pH 7.0 | 10 |
| | Wash 3 | 2.5 | 25 mM Phosphate, pH 7.0 | 10 |
| | line wash | 2.5 | 100 mM Citrate, pH 3.5 | 2 |
| | elute | 2.5 | 100 mM Citrate, pH 3.5 | 10 |
| | strip | 2.5 | 100 mMNaOH | 10 |
| | re-equilibration 1 | 2.5 | 100 mM Citrate, pH 3.5 | 4 |
| | re-equilibration 2 | 2.5 | 25 mM Phosphate, pH 7.0 | 10 |

**Example 2:** Additional breakthrough curves were generated with CHO cell culture harvest clarified by depth filtration and further pre-treated to remove impurities potentially responsible for the increased backpressure.

The clarified CHO cell culture harvest was again filtered using a Millipore, Durapore 0.45µm PVDF filter and then pre-treated by running it through a Natrix Recon NatriFlo HD-Q anion exchange adsorber (AEX) at 10 mL/min to remove impurities. A maximum of 40 mL of feed was processed before the AEX cartridge was stripped and regenerated. The AEX pre-treated feed was then used to generate new breakthrough curves for both the 1 mL and the 3.5 mL Protein A adsorbers using the same operating parameter used in Example 1.

**Figure 3**A & C show the overlays from the feeding step of two identical batch runs with and without the AEX pre-treatment. Figure 3A shows the results obtained with the 1 mL device and Figure 3C shows the results for the 3.5 mL device. The AEX pre-treatment resulted in a reduced backpressure increase over time and a lower final pressure value for both the devices demonstrating that the membrane volume did not influence the effectiveness of pre-treatment. Figure 3A shows that the specific backpressure increase is ΔPs = 0.051 bar*(mL adsorbent)/(mL feed) in the run without pre-treatment and ΔPs = 0.016 bar*(mL adsorbent)/(mL feed) in the run with AEX pre-treatment. Thus the specific backpressure increase is reduced by almost 70% by using pre-treatment. In Figure 3C, the specific backpressure increase is ΔPs = 0.075 bar*(mL adsorbent)/(mL feed) in the run without pre-treatment and ΔPs = 0.035 bar*(mL adsorbent)/(mL feed) in the run with AEX pre-treatment. In this case the specific backpressure increase is reduced more than 50% by use of the pre-treatment.

A different pre-treatment of clarified CHO cell culture harvest was carried out using the Natrix Recon HD-Sb cation exchange adsorber (CEX) at 10 mL/min to remove cationic impurities. Again a breakthrough curve was carried out using the 1 mL membrane-based Protein A device and Figure 3B shows the overlay of backpressures from the feed step with and without the CEX treatment. In the former case the specific backpressure increase is ΔPs = 0.051 bar*(mL adsorbent)/(mL feed) and in the latter case the specific backpressure increase is ΔPs = 0.040 bar*(mL adsorbent)/(mL feed). Thus, in this case the treatment is almost ineffective for backpressure increase reduction. All AEX and CEX pre-treatment and Protein A capture steps were carried out using a Contichrom CUBE Combined 30 system (ChromaCon AG, Switzerland).

**Example 3:** A twin-adsorber periodic countercurrent process was carried out using two membrane-based 3.5 mL Protein A devices (GORE Inc., DE, USA) with the operating parameters detailed in Table 4 (no AEX pre-treatment) and the Table 5 (with AEX pre-treatment). The clarified CHO cell culture harvest and buffers were filtered using a Millipore, Durapore 0.45µm PVDF membrane and half the supernatant was then pre-treated with a Natrix Recon NatriFlo HD-Q anion exchange adsorber (AEX) at 10 mL/min to remove impurities.

The first experiment, without AEX pre-treatment, was set up in order to establish if the increase in backpressure was reversible, in which case the backpressure would remain constant over the course of consecutive cycles, even if there was an increase during the feed step, as seen in examples 1 and 2. **Figure 4**A is a pressure profile of three cycles of the countercurrent process overlaid. **Figure 5**A shows a zoom of the pressure profiles from the first interconnected loading step. Both figures clearly demonstrate that, despite using the recommended of 100 mM NaOH strip step between cycles, the backpressure accumulates with each consecutive cycle and is only partially reversible. This phenomena caused one membrane device housing to burst on the fourth cycle showing that, without a pre-treatment, the Protein A membrane capture devices are unsuitable for continuous use in either repetitive batch or counter-current scenarios.

The second experiment was carried out with AEX pre-treated feed and results are shown in Figure 4B and Figure 5B. Both figures show cycles 4, 5 & 6 out of 6 total cycles. There is no increase in backpressure after 6 cycles demonstrating the effectiveness of the AEX pre-treatment to enable the use of the Protein A membrane devices for batch or PCC chromatography.

Figure 4C shows an example of a PCC process using particle-based packed-bed adsorbers (Eshmuno A, 0.8 cm inner diameter x 10 cm bed height) showing the backpressure increases from cycle to cycle. Thus resin based processes may also benefit from this type of pre-treatment. All AEX pre-treatment and Protein A capture steps were carried out using a Contichrom CUBE Combined 30 system (ChromaCon AG, Switzerland).

**Table 4: Periodic counter-current process operating parameters for the 3.5 mL membrane-based device run with clarified feed without further pre-treatment.**

| **Phase** | **step** | **Q_{buffer}** | **buffer** | **Q_{feed}** | **t** |
|---|---|---|---|---|---|
| **[-]** | **[-]** | **[mL/min]** | **[-]** | **[mL/min]** | **[min]** |
| **B** | wash | 18 | 25 mM Phosphate, 150 mM NaCl pH 7.0 | 1.215 | 1.94 |
| | elute | 11.781 | 100 mM Citrate, pH 3.5 | 1.215 | 2.97 |
| | neutralize | 18 | 25 mM Phosphate, 150 mM NaCl pH 7.0 | 1.215 | 0.39 |
| | strip | 7.5 | 100 mM NaOH | 1.215 | 2.33 |
| | Re-equilibrate | 18 | 25 mM Phosphate, 150 mM NaCl pH 7.0 | 1.215 | 1.55 |
| | wash line | 18 | 25 mM Phosphate, 150 mM NaCl pH 7.0 | | 0.39 |
| **IC** | feed | 0 | - | 11.781 | 4.75 |
| | wash | 11.781 | 25 mM Phosphate, 150 mM NaCl pH 7.0 | 0.0 | 2.97 |
| | | | | | |
| **Start-up** | feed | 0 | - | 11.781 | 0.91 |

**Table 5: Periodic counter-current process operating parameters for the 3.5 mL membrane-based device run with clarified feed with AEX pre-treatment.**

| **Phase [-]** | **step [-]** | **Q_{buffer} [mL/min]** | **buffer [-]** | **Q_{feed} [mL/min]** | **t [min]** |
|---|---|---|---|---|---|
| **B** | wash | 18 | 25 mM Phosphate, 150 mM NaCl pH 7.0 | 1.215 | 1.94 |
| | elute | 11.781 | 100 mM Citrate, pH 3.5 | 1.215 | 2.97 |
| | neutralize | 18 | 25 mM Phosphate, 150 mM NaCl pH 7.0 | 1.215 | 0.39 |
| | strip | 18 | 100 mM NaOH | 1.215 | 0.97 |
| | Re-equilibrate | 18 | 25 mM Phosphate, 150 mM NaCl pH 7.0 | 1.215 | 1.55 |
| | wash line | 18 | 25 mM Phosphate, 150 mM NaCl pH 7.0 | | 0.39 |
| **IC** | feed | 0 | - | 11.781 | 4.75 |
| | wash | 11.781 | 25 mM Phosphate, 150 mM NaCl pH 7.0 | 0.0 | 2.97 |
| | | | | | |
| **Start-up** | feed | 0 | - | 11.781 | 0.91 |

**Example 4:** Figure 6A shows the batch chromatogram overlay (UV A280 nm) comparing the breakthrough and elution profile with and without the AEX pre-treatment. Operating conditions were those described in Example 1 - Table 2. Here it is evident that, while the elution peaks are of the same height (non-normalized), there is clearly a reduction in the impurities peak that corresponds to the AEX treatment (dashed line). Figure 6B shows a size exclusion HPLC (SE-HPLC) chromatogram of the feed material with and without AEX pre-treatment and again the major peak corresponding to the mAb is the same size (non-normalized). However, there is a reduction in molecular weight species both above and below the mAb product after AEX treatment. Both results confirm that the AEX treatment removes impurity species while the amount of IgG in the product is not affected by the pre-treatment.

The equivalent batch chromatogram overlay comparison for CEX treatment in Figure 6C (UV A280 nm) also shows some removal of impurities, but to a lesser degree. The SE-HPLC chromatogram in Figure 6D shows that, compared to AEX treatment, there are many impurities still present. Moreover the results show that a fraction of the IgG is removed through the CEX treatment, which is not detrimental to the overall process yield. All AEX / CEX pre-treatment and Protein A capture steps were carried out using a Contichrom CUBE Combined 30 system (ChromaCon AG, Switzerland).

### LIST OF REFERENCE SIGNS

| | | | |
|---|---|---|---|
| AEX | anion exchange | PCC | periodic counter-current |
| B | batch step/phase | PVDF | Polyvinylidene Fluoride |
| CEX | cation exchange | SE-HPLC | size exclusion high pressure liquid chromatography |
| CHO cells | Chinese hamster ovary cells | | |
| Fc | Fc protein domain | Lₛ | specific loading |
| HCP | host cell protein | V_{feed} | load of feed |
| IC | interconnected step/phase | V_{ads} | adsorbent volume |
| B1 | first batch phase | t_{feed} | feed period |
| I1 | first interconnected phase | Q_{feed} | volumetric feed flow rate |
| B2 | second batch phase | ΔP | backpressure difference |
| 12 | second interconnected phase | ΔPs | specific backpressure increase |
| IgG | Immunoglobulin G | | |

## Claims

1. A method for purification of at least one protein target compound from a cell culture harvest comprising said at least one protein target compound as well as impurities, said method comprising the following steps in given order:
a) at least one step for removal of insoluble impurities from the initial cell culture harvest;
b) at least one further initial cell culture harvest pre-treatment step for removal of anionic soluble impurities from the cell culture harvest;
c) separation of the at least one protein target compound from the cell culture harvest by means of affinity chromatography,
with the proviso that the order steps a) and b) can be reversed or that the steps a) and b) are carried out in one device,
and with the proviso that further pre-treatment steps can be carried out before the terminal step c) of affinity chromatography separation.

2. Method according to claim 1, wherein said affinity chromatography separation step c) comprises at least two different phases (IC, B),
at least one interconnected phase (IC), in which two affinity chromatography adsorbers are interconnected in that the outlet of an upstream adsorber is fluidly connected to the inlet of a downstream adsorber, and
at least one batch phase (B), in which at least one affinity chromatography adsorber is not fluidly connected to the others and in which the at least one protein target compound is recovered in purified form from a disconnected adsorber.

3. Method according to any of the preceding claims, wherein the step a) for removal of insoluble impurities from the initial cell culture harvest, or at least one module of a corresponding pre-treatment device, includes filtration, centrifugation, flocculation steps, acoustic separation, or precipitation steps, or a combination of these aforementioned techniques and wherein the filtration techniques include depth filtration, absolute filtration or ultrafiltration.

4. Method according to any of the preceding claims, wherein the steps a) for removal of insoluble impurities from the initial cell culture harvest, as well as the step b) for removal of anionic soluble impurities, are carried out by the same medium in one step, in that the method or at least one module of a corresponding pre-treatment device, includes filtration, centrifugation, flocculation steps, acoustic separation, or precipitation steps, or a combination of these aforementioned techniques and wherein the filtration techniques include depth filtration, absolute filtration or ultrafiltration, and wherein the corresponding method involves at the same time removal of anionic soluble impurities from the cell culture harvest, wherein preferably the method involves a filter carrying positive stationary charges.

5. Method according to any of the preceding claims, wherein step b) is performed using a chromatographic sorbent for removal of anionic soluble impurities and wherein preferably the sorbent is based on membranes, packed beds of particles, inorganic compounds, monoliths, fibers or combinations thereof
and/or wherein step b) uses an anion exchange functionality, or is based on hydroxyapatite or fluoroapatite and derivatives thereof.

6. Method according to any of the preceding claims, wherein step c) is performed using an affinity chromatography sorbent based on membranes, packed beds of particles, monoliths, fibers, inorganic compounds, or combinations thereof,
and/or wherein step c) is performed using an affinity chromatography sorbent for protein A, protein G, protein L, metal ion affinity chromatography, organic ligands, heparin, or affinity chromatography using other proteinaceous or peptidic ligands.

7. A method according to any of the preceding claims wherein in the affinity chromatography step c) uses at least two affinity chromatography adsorbers, preferably of the same type, preferably exactly two affinity chromatography absorbers,
and wherein preferably in the affinity chromatography step c) comprises at least two cycles wherein each cycle includes at least one interconnected phase (IC) wherein the outlet of an upstream adsorber is connected to the inlet of a downstream adsorber, as well as at least one batch phase (B) in which the adsorbers are not fluidly connected, and wherein preferably the affinity chromatography step c) is operated over at least two cycles.

8. Method according to any of the preceding claims, wherein in the affinity chromatography step c) is carried out using at least two affinity chromatography adsorbers, and comprises at least four different steps (IC1, B1, IC2, B2) in given order, within one cycle to be carried out at least once, preferably a plurality of times, preferably using only two affinity chromatography adsorbers:
a first batch step (Bl), wherein during a batch timespan (t_{B}) said adsorbers are disconnected and
a first adsorber is loaded with feed with the cell culture harvest pretreated in steps a) and b) via its inlet, using a first flow rate (Q_{feed,B}) and its outlet is directed to waste, and
from a second adsorber the at least one protein target compound is recovered via its outlet and subsequently the second adsorber is regenerated;
a first interconnected step (IC1), wherein the outlet of the first adsorber is connected to the inlet of the second adsorber during an interconnected timespan (t_{IC}),
the first adsorber is loaded beyond its dynamic breakthrough capacity with feed via its inlet using a second flow rate (Q_{feed,IC}) which is the same or larger than the first flow rate (Q_{fecd,B}), and
the outlet of the second adsorber is directed to waste,
and wherein during a subsequent washing timespan (t_{wash,IC}) which is larger or equal to 0 s
the outlet of the first adsorber is connected to the inlet of the second adsorber,
the first adsorber is loaded with solvent and/or buffer which is free from feed material, and
the outlet of the second adsorber is directed to waste,
a second batch step (B2) analogous to the first batch step (B1) but with exchanged adsorber positions, such that the first adsorber of the first batch step (B1) performs the tasks of the second adsorber of the first batch step (B1), and the second adsorber of the first batch step (B1) performs the tasks of the first adsorber of the first batch step (B1);
a second interconnected step (IC2), analogous to the first interconnected step (IC1) but with exchanged adsorber positions, such that the upstream adsorber of the first interconnected step (IC1) is the downstream adsorber of the second interconnected step (IC2), and the downstream adsorber of the first interconnected step (B1) is the upstream adsorber of the second interconnected step (IC2).

9. Method according to any of the preceding claims, wherein the protein target compound is a monoclonal antibody, bispecific antibody, antibody fragment, multispecific antibody framework, antibody scaffolds, or fusion protein.

10. A method according to any of the preceding claims wherein step b) includes a regeneration step and the device is operated in a cyclic manner.

11. A method according to any of the preceding claims wherein step c) is followed by a virus inactivation step
and/or a method using at least two chromatographic non-affinity adsorbers as chromatographic stationary phase, grouped into only one first adsorber section and one second adsorber section,
wherein if an adsorber section comprises more than one chromatographic adsorber these are permanently connected in series,
wherein the first adsorber section has a first adsorber section inlet and a first adsorber section outlet, and the second adsorber section has a second adsorber section inlet and a second adsorber section outlet,
the method comprising the following steps in order:
a. a first interconnected step (IC1a), wherein the first adsorber section outlet is connected to the second adsorber section inlet during an first interconnected timespan (t_{ICa}), wherein the first adsorber section (1) is loaded via the first adsorber section inlet with feed mixture and wherein product is collected from the second adsorber section outlet;
b. a second interconnected step (IC1b), wherein the first adsorber section outlet is connected to the second adsorber section inlet during a second interconnected timespan (t_{ICb}), wherein the first adsorber section is loaded via the first adsorber section inlet with a first washing buffer to transfer product unbound in the first absorber section into the second adsorber section and wherein the stream exiting the first adsorber section outlet is
either diluted inline before entering the second adsorber section inlet or is supplemented with feed mixture
and wherein product is collected from the second adsorber section outlet;
c. a first batch step (B1), wherein during a batch timespan (t_{B}) said first and second adsorber sections are disconnected and
wherein the first adsorber section is cleaned and regenerated to remove impurities and the first adsorber section outlet is directed to waste and wherein the second adsorber section inlet is
either loaded with a second washing buffer
or loaded with feed mixture
and product is collected from the second adsorber section outlet;
d. a third interconnected step (IC2a), wherein the first adsorber section performs tasks of the second adsorber section in the first interconnected step, and the second adsorber section performs tasks of the first adsorber section in the first interconnected step;
e. a fourth interconnected step (IC2b), wherein the first adsorber section performs tasks of the second adsorber section in the second interconnected step, and the second adsorber section performs tasks of the first adsorber section in the second interconnected step;
f. a second batch step (B2), wherein the first adsorber section performs tasks of the second adsorber section in the first batch step, and the second adsorber section performs tasks of the first adsorber section in the first batch step.

12. A method according to any of the preceding claims, wherein the pre-treatment step b) leads to a reduction of the specific backpressure increase (ΔPs) in the subsequent affinity step c) by at least 50%, preferably by at least 60%, or by at least 70%, most preferably by at least 80%, in each case relative to the specific backpressure increase without such pre-treatment step b),
and/or wherein the pre-treatment step b) leads to a reduction of the specific backpressure increase (ΔPs) in the subsequent affinity step c) by at least 0.02 bar*(mL adsorbent)/(mL feed), preferably by at least 0.03 bar*(mL adsorbent)/(mL feed), most preferably by least 0.04 bar*(mL adsorbent)/(mL feed).

13. Use of a pre-treatment for removal of anionic soluble impurities from a cell culture harvest for reducing the back pressure in a subsequent affinity step capture separation.

14. Use according to claim 13, wherein for purification of at least one protein target compound from a cell culture harvest comprising said at least one protein target compound as well as impurities, the following steps are carried out in given order:
a) at least one step for removal of insoluble impurities from the initial cell culture harvest;
b) at least one further initial cell culture harvest pre-treatment step for removal of anionic soluble impurities from the cell culture harvest;
c) separation of the at least one protein target compound from the cell culture harvest by means of affinity chromatography,
with the proviso that the order steps a) and b) can be reversed or that the steps a) and b) are carried out in one device in one step,
and with the proviso that further pre-treatment steps can be carried out before the terminal step c) of affinity chromatography separation.

15. Use according to any of claims 13 or 14, wherein the removal of anionic soluble impurities is carried out by anionic exchange chromatography (AEX), preferably by using a packed particle bed or a membrane anionic exchange chromatography device or a charged depth filter.
